# EUROPEAN PATENT APPLICATION

(11) **EP 0 699 675 A2**
(43) Date of publication of application: **06.03.1996**
(21) Application number: 95113757.9
(22) Date of filing: 01.09.1995
(51) Int. Cl.: C07D 417/00, A61K 31/425

(54) **3-(4-(1-Substituted-4-piperazinyl)butyl)-4-thiazolidinone and related compounds**

(30) Priority: 01.09.1994 US 299880
(71) Applicant: HOECHST-ROUSSEL PHARMACEUTICALS INCORPORATED, Somerville, NJ 08876-1258 (US)
(72) Inventor: Hrib, Nicolas Joseph, Somerville, New Jersey 08876 (US); Jurcak, John Gerard, Union City, New Jersey 07087 (US)
(74) Representative: Losert, Wolfgang Dr.

(57) **Abstract**

There are disclosed compounds of the formula, 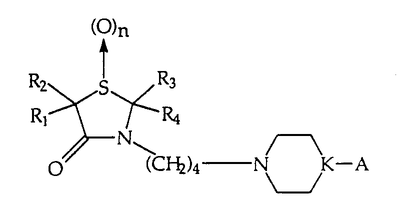
where n is 0, 1 or 2; A is 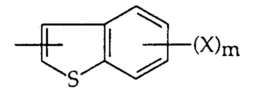
where X in each occurrence is independently hydrogen, halogen, loweralkyl, hydroxy, nitro, loweralkoxy, amino, cyano, trifluoromethyl or methylthio; Y in each occurrence is independently hydrogen, halogen, loweralkyl, hydroxy, nitro, loweralkoxy, amino, cyano, trifluoromethyl or methylthio; K is N or CH; m is 1 or 2; k is 1 or 2; R₁ and R₂ are independently hydrogen, loweralkyl, 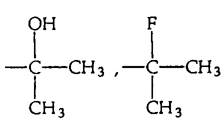
or aryl except that when R₁ is 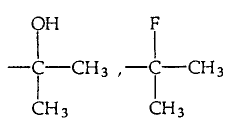
or aryl, R₂ is hydrogen, or alternatively R₁ + R₂ taken together with the carbon atom to which they are attached form a cyclopentane, cyclopentane, cyclohexane, cycloheptane, pyran, thiopyran, indan or piperidine ring; R₃ and R₄ are independently hydrogen or loweralkyl, or alternatively R₃ + R₄ taken together with the carbon atom to which they are attached form a cyclopentane, cyclohexane, cycloheptane, pyran, thiopyran, pyrrolidine or piperidine ring, the term aryl signifying an unsubstituted phenyl group or a phenyl group substituted with 1, 2 or 3 substituents each of which being independently loweralkyl, loweralkoxy, hydroxy, halogen, loweralkylthio, cyano, amino or trifluoromethyl, which are useful as antipsychotic, analgesic, anticonvulsant and anxiolytic agents.

## Description

The present invention relates to compounds of the formula 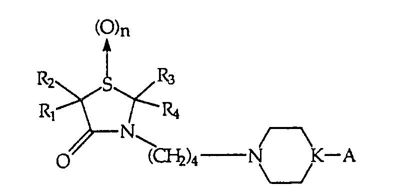
where n is 0, 1 or 2; A is 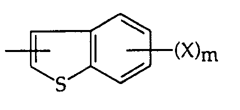
where X in each occurrence is independently hydrogen, halogen, loweralkyl, hydroxy, nitro, loweralkoxy, amino, cyano, trifluoromethyl or methylthio; Y in each occurrence is independently hydrogen, halogen, loweralkyl, hydroxy, nitro, loweralkoxy, amino, cyano, trifluoromethyl or methylthio; K is N or CH; m is 1 or 2; k is 1 or 2; R₁ and R₂ are independently hydrogen, loweralkyl, 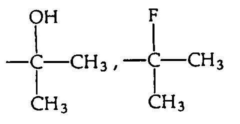
or aryl except that when R₁ is 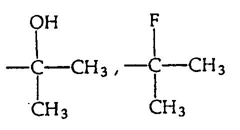
or aryl, R₂ is hydrogen, or alternatively R₁ + R₂ taken together with the carbon atom to which they are attached form a cyclopentane, cyclohexane, cycloheptane, pyran, thiopyran, indan or piperidine ring; R₃ and R₄ are independently hydrogen or loweralkyl, or alternatively R₃ + R₄ taken together with the carbon atom to which they are attached form a cyclopentane, cyclohexane, cycloheptane, pyran, thiopyran, pyrrolidine or piperidine ring, the term aryl signifying an unsubstituted phenyl group or a phenyl group substituted with 1, 2 or 3 substituents each of which being independently loweralkyl, loweralkoxy, hydroxy, halogen, loweralkylthio, cyano, amino or trifluoromethyl, which are useful as antipsychotic, analgesic, anticonvulsant and anxiolytic agents.

Throughout the specification and the appended claims, a given chemical formula or name shall encompass all stereo, geometrical and optical isomers thereof where such jsomers exist, as well as pharmaceutically acceptable acid addition salts thereof and solvates thereof such as, for instance, hydrates.

The following general rules of terminology shall apply throughout the specification and the appended claims.

Unless otherwise stated or indicated, the term loweralkyl denotes a straight or branched alkyl group having from 1 to 6 carbon atoms. Examples of said loweralkyl include methyl, ethyl, n-propyl, iso-butyl, pentyl and hexyl.

Unless otherwise states or indicated, the term cycloalkyl denotes an alicyclic hydrocarbon group containing 3 to 7 carbon atoms.

Unless otherwise stated or indicated, the term loweralkoxy denotes a straight or branched alkoxy group having from 1 to 6 carbon atoms. Examples of said loweralkoxy include methoxy, ethoxy, n-propoxy, iso-propoxy, n-butoxy, iso-butoxy, sec-butoxy, t-butoxy and straight- and branched-chain pentoxy and hexoxy.

Unless otherwise stated or indicated, the term halogen shall mean fluorine, chlorine, bromine or iodine.

Unless otherwise stated or indicated, the term aryl shall mean a phenyl group having 0, 1, 2 or 3 substituents each of which being independently loweralkyl, loweralkoxy, halogen, loweralkylthio, cyano, amino or CF₃.

The compounds of this invention are prepared by utilizing one or more of the steps described below. Throughout the description of the synthetic steps, the definitions of n, m, k, A, K, X, Y and R₁ through R₄ are as given above unless otherwise stated or indicated.

### STEP A

A compound of Formula II is reacted with 1,4-dibromobutane to afford a compound of Formula III.

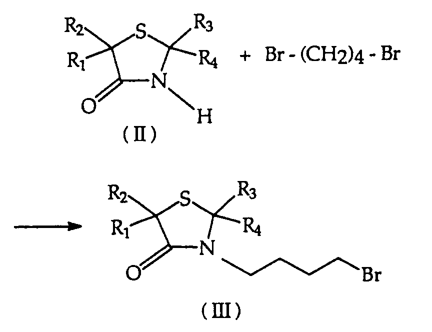

The above reaction is typically conducted in the presence of a suitable medium such as dimethylformamide or THF and a base such as potassium hydroxide, sodium hydroxide or sodium hydride at a temperature of about 23 to 70°C.

### STEP B

Compound III is reacted with a compound of Formula IV to afford a compound of Formula V. 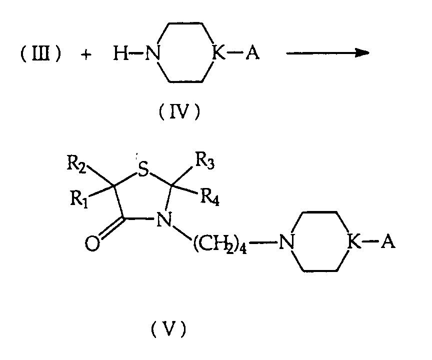
The above reaction is typically conducted in the presence of a suitable medium such as anhydrous acetonitrile, an acid scavenger such as potassium carbonate or sodium carbonate and a small amount of potassium iodide or sodium iodide at a temperature of about 20 to 100°C.

### STEP C

Compound V is oxidized with a suitable oxidizing agent such as NaIO₄ to afford a compound of Formula VI.

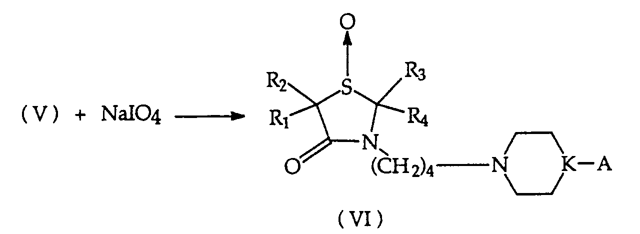

The above reaction is typically conducted in the presence of a suitable medium such as tetrahydrofuran and water at a temperature of about -10 to 23°C.

### STEP D

Compound III is oxidized in substantially the same manner as in STEP C to afford a compound of Formula VII.

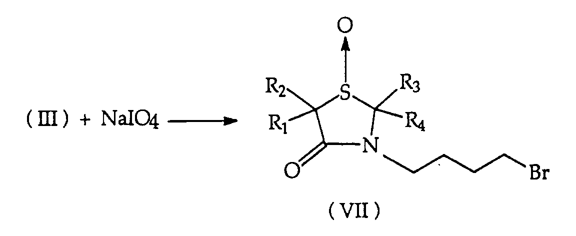

### STEP E

Compound VII is reacted with compound IV in substantially the same manner as in STEP B to afford a compound of Formula VI.

(VII) + (IV) → (VI)

### STEP F

As an alternative to the foregoing scheme, one can obtain a compound of Formula VIII where P is hydrogen, loweralkyl, loweralkoxy, hydroxy, loweralkylthio or amino by reacting a compound of Formula IX with an aromatic compound of Formula X. 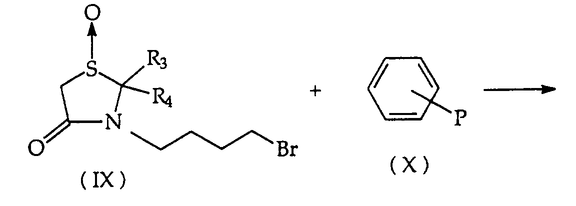 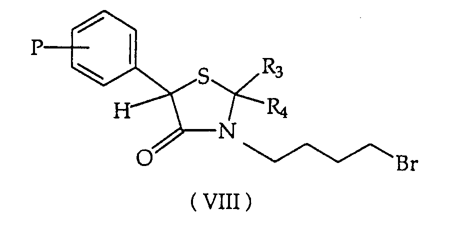
The above reaction is typically conducted in the presence of H₂SO₄ or p-toluenesulfonic acid at a temperature of about -10 to about 83°C.

### STEP G

As an alternative to the foregoing scheme, one can obtain a compound of Formula XI where the divalent group - R - plus the spiro carbon as combined constitutes a cyclopentane, cyclohexane, cycloheptane, pyran, thiopyran, indan or piperidine ring, in the following manner.

First, 4-thiazolidinone is reacted with t-butyldimethylsily chloride in a suitable solvent such as dichloromethane at a suitable temperature such as about 20-30°C to afford a mixture of compounds of Formulas XII and XIII. Typically, the molar ratio between compound XII and compound XIII is about 70:30.

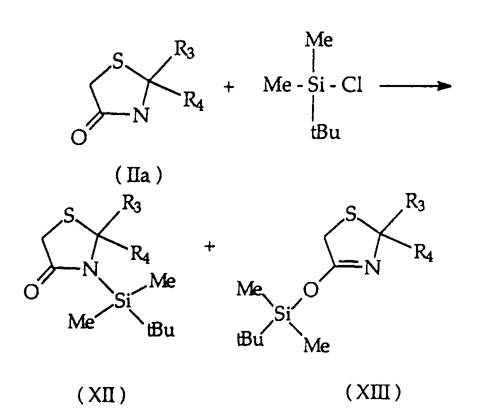

The above-mentioned mixture is reacted with lithium bis(trimethyl-silyl)amide and a compound of Formula XIV where R is as defined above and Hal is Br or I in a suitable medium such as tetrahydrofuran and at a low temperature such as -75°C to -50°C to afford Compound XI. 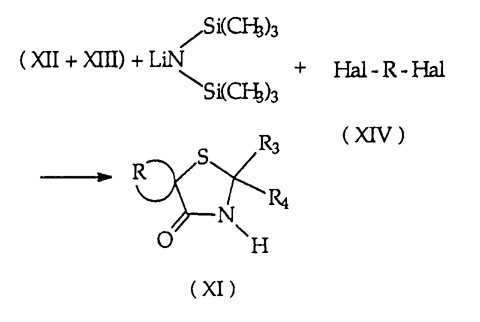
Similarly, if one uses a mono-bromide or mono-iodide of the Formula R₅ - Hal where R₅ is loweralkyl in the place of Hal - R - Hal, one can obtain a compound of Formula XV and/or XVI. In this reaction, if one desires to obtain compound XV as a predominant product, it is preferable to adjust the molar ratio between compound IIa and lithium bis(trimethylsilyl)amide to about 1:1, whereas if one desires to obtain compound XVI as a predominant product, it is preferable to adjust said molar ratio to about 1:2, also making a judicious choice in the amount of the halide compound used in each case. The two products can easily be separated from each other with the aid of a routine technique such as chromatography.

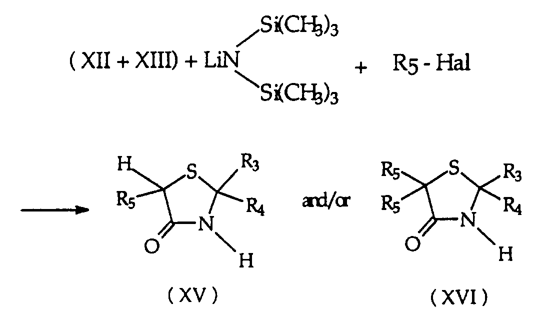

### STEP H

Compound IIIa (R₁ = R₂ = H) is allowed to react with lithium bis(trimethylsily)amide and compound XIV in substantially the same manner as in STEP G to afford a compound of formula XVII. 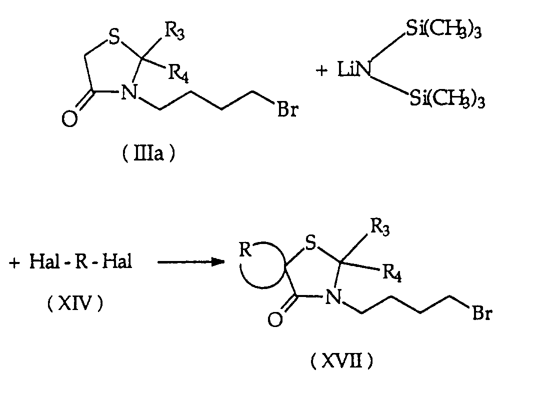
Similarly, if one uses R₅ - Hal instead of Hal - R - Hal, one can obtain a compound of formula XVIII and/or XIX. In this reaction, if one desires to obtain compound XVIII as a predominant product, it is preferable to adjust the molar ratio between compound IIIa and lithium bis(trimethylsilyl)amide to about 1:1, whereas if one desires to obtain compound XIX as a predominant product, it is preferable to adjust said molar ratio to about 1:2, also making a judicious choice in the amount of the halide compound used in each case. The two products can easily be separated from each other with the aid of a routine technique such as chromatography.

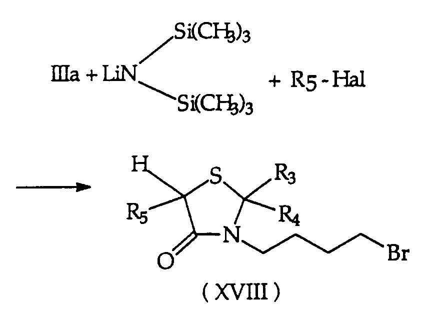

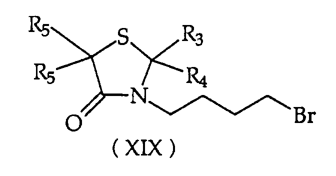

### STEP I

Compound IIIa is allowed to react with lithium bis(trimethylsilyl)amide and about three equivalents of acetone in substantially the same manner as in STEP G to afford a compound of Formula XX.

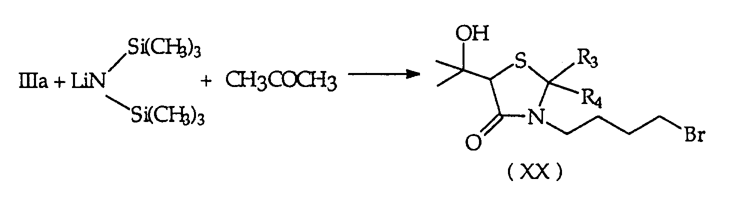

### STEP J

Compound XX is allowed to react with dimethylaminosulfur trifluoride to afford a compound of Formula XXI. 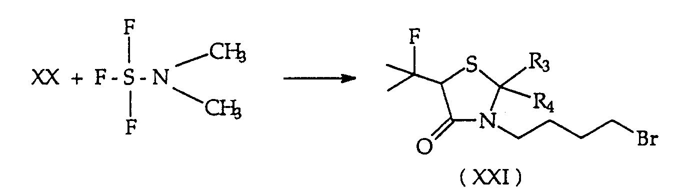
The above reaction is typically conducted in a suitable solvent such as dichloromethane at a temperature of -78 to 0°C.

### STEP K

Compound III is oxidized with a suitable oxidizing agent such as potassium peroxymonosulfate (2KHSO₅·KHSO₄·H₂SO₄) to afford a compound of Formula XXIII. 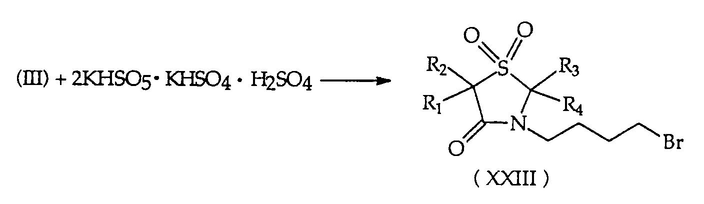
The above reaction is typically conducted in the presence of a suitable medium such as water and ethanol at a temperature of about -10 to 25°C.

### STEP L

Compound XXIII is reacted with compound IV in substantially the same manner as in STEP B to afford a compound of Formula XXII. 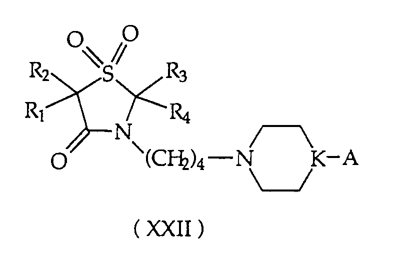
The compounds of the present invention having Formula I are useful as antipsychotic agents.

Antipsychotic activity is determined in the climbing mice assay by methods similar to those described by P. Protais, et al., Psychopharmacol., 50, 1 (1976) and B. Costall, Eur. J. Pharmacol., 50, 39 (1978).

The subject CK-1 male mice (23-27 grams) are group-housed under standard laboratory conditions. The mice are individually placed in wire mesh stick cages (4" x 4" by 10") and are allowed one hour for adaptation and exploration of the new environment. Then apomorphine is injected subcutaneously at 1.5 mg/kg, a dose causing climbing in all subjects for 30 minutes. Compounds to be tested for antipsychotic activity are injected intraperitoneally 30 minutes prior to the apomorphine challenge at a screening dose of 10mg/kg.

For evaluation of climbing, 3 readings are taken at 10,20 and 30 minutes after apomorphine administration according to the following scale:

| Climbing Behavior Mice with: | Score |
|---|---|
| 4 Paws on bottom (no climbing) | 0 |
| 2 paws on the wall (rearing) | 1 |
| 4 paws on the wall (full climb) | 2 |

Mice consistently climbing before the injection of apomorphine are discarded.

With full-developed apomporphine climbing, the animals are hanging onto the cage walls, rather motionless, over longer periods of time. By contrast, climbs due to mere motor stimulation usually last only a few seconds.

The climbing scores are individually totaled (maximum score: 6 per mouse over 3 readings) and the total score of the control group (vehicle intraperitoneally-apomorphine subcutaneously) is set to 100%. The results are presented for some compounds in Table 1. ED₅₀ value with 95% confidence limits, calculated by a linear regression analysis of some of the compounds of this invention.

**TABLE 1**

| Compound | Antipsychotic Activity Dose mg/kg ip | Score |
|---|---|---|
| 3-[4-[4-(Benzo[b]thien-2-yl)-1-piperidinyl]butyl]2.5.5-trimethyl-4-thiazolidinone | 20 | 44% |
| 3-[4-[4-(Benzo[b]thien-3-yl)-1-piperidinyl]butyl]-2.5.5-trimethyl-4-thiazolidinone maleate | 9.5 | 50% |
| 3-[4-[4-(Benzo[b]thien-3-yl)-1-piperidinyl]butyl]-5,5-dimethyl-1,1-dioxo-4-thienzolidinone | 20 | 98% |

Effective quantities of the compound of the invention may be administered to a patient by any of the various methods, for example, orally as in capsule or tablets, parenterally in the form of sterile solutions or suspensions, and in some cases intravenously in the form of sterile solutions. The free base final products, while effective themselves, may be formulated and administered in the form of their pharmaceutically acceptable acid addition salts for purposes of stability, convenience of crystallization, increased solubility and the like.

Acids useful for preparing the pharmaceutically acceptable acid addition salts of the invention include inorganic acids such as hydrochloric, hydrobromic, sulfuric, nitric, phosphoric and perchloric acids, as well as organic acids such as tartaric, citric, acetic, succinic, maleic, fumaric and oxalic acids.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compounds, but may be varied depending upon the particular form and may conveniently be between 5% to about 70% of the weight of the unit. The amount of active compound in such compositions is such that a suitable dosage will be obtained. Preferred compositions and preparations according to the present invention are prepared so that an oral dosage unit form contains between 1.0-3.00 milligrams of active compound.

The tablets, pills, capsules, troches and the like may also contain the following ingredients: a binder such as micro-crystalline cellulose, gum tragacanth or gelatin; an excipient such as starch or lactose, a disintegrating agent such as alginic acid, Primogel, cornstarch and the like; a lubricant such as magnesium stearate or Sterotex; a glidant such as colloidal silicon dioxide; and a sweeting agent such as sucrose or saccharin may be added or a flavoring agent such as peppermint, methyl salicylate, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes, coloring and flavors. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0.1% of active compound, but may be varied between 0.5 and about 30% of the weight thereof. The amount of active compound in such compositions is such that a suitable dosage will be obtained. Preferred compositions and preparations according to the present inventions are prepared so that a parenteral dosage unit contains between 0.5 to 100 milligrams of active compound.

The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates; citrates or phosphates and agents for the adjustment of tonicity such as sodium chloride or dextrose. The parenteral multiple vials may be of glass or plastic.

Examples of the compounds of this invention include:
3-[4-[4-(benzo[b]-thien-2-yl)-1-piperidinyl]butyl]-2,5,5-trimethyl-4-thiazolidinone hydrochloride;
3-[4-[4-(benzo[b]thien-3-yl)-1-piperidinyl]butyl]-2,5,5-trimethyl-4-thiazolidinone maleate; and
3-[4-[4-(benzo[b]-thien-3-yl)-1-piperidinyl[butyl]-5,5-dimethyl-1,1-dioxo-4-thiazolidinone.

The following examples are presented in order to illustrate this invention.

### EXAMPLE 1

### 3-(4-Bromobutyl)-2,5,5-trimethyl-4-thiazolidinone

To a -73°C solution of 3-(4-bromobutyl)-2-methyl-4-thiazolidinone (6.00 g) methyliodide (10.99 g) and THF (50 ml) under nitrogen was added lithium bis(trimethylsilyl)amide (0.0500 mol) in THF (50 ml) at a rate to maintain the internal temperature below -55°C. The resulting solution was stirred at, -55°C for 10 minutes, allowed to warm to -40°C, and at which temperature 1 N HCl (250 ml) was added. The aqueous mixture was extracted with 25% benzene/ether (3 x 125 ml). The combined extracts were washed with brine (200 ml), dried (Na₂SO₄), and concentrated to a liquid which was chromatography on silica gel yielding 5.07 g of a liquid. The liquid was distilled to give 3.80 g of a clear liquid, b.p. 109-114°C at 0.20 mmHg.

| ANALYSIS: | | | |
|---|---|---|---|
| Calculated for C₁₀H₁₈BrNOS: | 42.86%C | 6.47%H | 5.00%N |
| Found: | 42.93%C | 6.47%H | 5.00%N |

### EXAMPLE 2

### 3-(4-Chlorobutyl)-2,5,5-trimethyl-1,1-dioxo-4-thiazolidinone

A suspension of potassium peroxymonosulfate (32.0 g) and water (120 mL) was added to a 2°C solution of 3-(4-chlorobutyl)-2,5,5-trimethyl-4-thiazolidinone (8.0 g) and ethanol (80 mL) over a period of 30 minutes. The cold bath was removed and the resulting mixture was stirred at room temperature for 22 hours, after which ethyl acetate (200 mL) was added. The mixture was filtered, the insolubles washed with ethyl acetate (3 x 30 mL), and the combined filtrates were diluted with water (200 mL). The organic layer was separated, and the aqueous layer extracted with ethyl acetate (2 x 100 mL). The combined organic extracts were washed with saturated aqueous NaHCO₃ (200 mL), water (200 mL), and brine (200 mL), dried (Na₂SO₄), and concentrated *in vacuo* to 8.62 g of an off-white solid. The sulfone was recrystallized from ether to give 5.67 g (62.4%) of a white crystalline solid, m.p. 71-73°C.

| ANALYSIS: | | | |
|---|---|---|---|
| Calculated for C₁₀H₁₈ClNO₃S: | 44.86%C | 6.78%H | 5.23%N |
| Found: | 44.71%C | 6.67%H | 5.20%N |

### EXAMPLE 3

### 3-[4-[4-[Benzo[b]thien-2yl)-1-piperidinyl[butyl]-2,5,5-trimethyl-4-thiazolidinone hydrochloride

A mixture of 4-(benzo[b]thien-2-yl)-piperidine (3.00 g, 13.8 mmol), 3-(4-chlorobutyl)-2,5,5-trimethyl-4-thiazolidinone (4.07 g, 17.3 mmol), K₂CO₃ (2.86 g, 20.7 mmol), KI (0.68 g), and 90% aqueous acetonitrile (100 mL) was refluxed for 88 hours. The solvent was removed *in vacuo*, the residue treated with 5% NaOH (250 mL), and the aqueous mixture extracted with dichloromethane (3 x 100 mL). The combined extracts were washed with water (150 mL), dried (K₂CO₃), and concentrated *in vacuo* to a brown liquid. The crude product was purified by chromatography on silica gel, eluting with 10% methanol in dichloromethane, to afford 4.84 g of the product as an oil. The hydrochloride salt was prepared using ethanolic hydrogen chloride and recrystallized from ethanolethyl acetate to yield 1.73 g (28%) crystals, m.p. 196-198°C.

| ANALYSIS: | | | |
|---|---|---|---|
| Calculated for C₂₃H₃₂N₂OS·HCl: | 60.97%C | 7.34%H | 6.18%N |
| Found: | 60.80%C | 7.28%H | 6.04%N |

### EXAMPLE 4

### 3-[4-[4-(Benzo[b]thien-3-yl)-1-piperidinyl]butyl]-2,5,5-trimethyl-4-thiazolidinone maleate

A mixture of 4-(benzo[b]thien-3-yl)-piperidine (3.30 g, 15.2 mmol), 3-(4-cholorobutyl)-2,5,5-trimethyl-4-thiazolidinone (4.48 g, 19.0 g mmol), K₂CO₃ (3.15 g, 22.8 mmol), KI (0.75 g), and 90% aqueous acetonitrile (100 mL) was refluxed for 48 hours. The solvent was removed *in vacuo*, the residue treated with 5% NaOH (250 mL), and the aqueous mixture extracted with dichloromethane (2 x 100 mL). The combined extracts were washed with water (100 mL), dried (K₂CO₃), and concentrated *in vacuo* to a brown liquid. The crude product was purified by chromatography on silica gel, eluting with 20% methanol in ethyl acetate to afford 4.38 g of the product as an oil. To a solution of the free base (3.01 g, 7.22 mmol) in ethanol was added maleic acid (0.880 g, 7.58 mmol) and the mixture was heated until a solution was obtained. The solvent was removed *in vacuo* and the salt recrystallized from methanol/ethyl acetate to yield 2.89 g (36%) of the product as flakes, m.p. 165-166°C.

| ANALYSIS: | | | |
|---|---|---|---|
| Calculated for C₂₃H₃₂N₂OS₂·C₄H₄O_{4:} | 60.88%C | 6.81%H | 5.26%N |
| Found: | 61.15%C | 6.51%H | 5.23%N |

### EXAMPLE 5

### 3-[4-[4-[Benzo[b]thien-3-yl)-1-piperidinyl]butyl]-5,5-dimethyl-1,1-dioxo-4-thiazolidinone

A mixture of 4-(benzo[b]thien-3-yl)-piperidine (4.16 g, 19.1 mmol), 3-(4-chlorobutyl)-5,5-dimethyl-1,1-dioxo-4-thiazolidinone (4.00 g, 15.8 mmol), K₂CO₃ (3.50 g, 25.3 mmol), KI (0.75 g), and 90% aqueous acetonitrile (100 mL) was refluxed for 30 hours. The mixture was diluted with water (50 mL) and the solution concentrated *in vacuo*. The residue was treated with 5% aqueous NaOH (200 mL), and the mixture extracted with dichloromethane (2 x 100 mL). The combined extracts were washed with water (100 mL), dried (K₂CO₃), and concentrated *in vacuo*. The crude product was purified by chromatography on silica gel, eluting with 10% methanol in dichloromethane, to afford 5.00 g of the product as a viscid material which gradually solidified to a white solid. The free base was recrystallized from ethyl acetate/heptane to yield 2.34 g (34%) of the product, m.p. 102-104°C.

| ANALYSIS: | | | |
|---|---|---|---|
| Calculated for C₂₂H₃₀N₂O₃S₂: | 60.80%C | 6.96%H | 6.45%N |
| Found: | 60.57%C | 6.75%H | 6.37%N |

## Claims

1. A compound of the formula 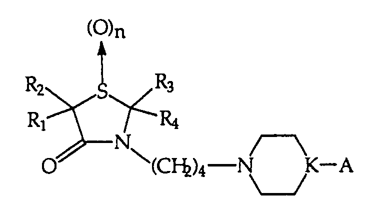 where n is 0,1 or 2; A is 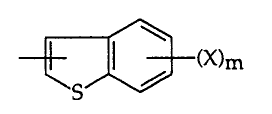 where X in each occurrence is independently hydrogen, halogen, loweralkyl, hydroxy, nitro, loweralkoxy, amino, cyano, trifluoromethyl or methylthio; Y in each occurrence is independently hydrogen, halogen, loweralkyl, hydroxy, nitro, loweralkoxy, amino, cyano, trifluoromethyl or methylthio; K is CH; m is 1 or 2; k is 1 or 2; R₁ and R₂ are independently hydrogen, loweralkyl, 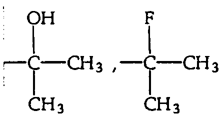 or aryl except that when R₁ is 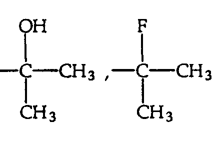 or aryl, R₂ is hydrogen, or alternatively R₁ + R₂ taken together with the carbon atom to which they are attached form a cyclopentane, cyclopentane, cyclohexane, cycloheptane, pyran, thiopyran, indan or piperidine ring; R₃ and R₄ are independently hydrogen or loweralkyl, or alternatively R₃ + R₄ taken together with the carbon atom to which they are attached form a cyclopentane, cyclohexane, cycloheptane, pyran, thiopyran, pyrrolidine or piperidine ring, the term aryl signifying an unsubstituted phenyl group or a phenyl group substituted with 1, 2 or 3 substituents each of which being independently loweralkyl, loweralkoxy, hydroxy, halogen, loweralkylthio, cyano, amino or trifluoromethyl, or a pharmaceutically acceptable acid addition salt thereof.

2. The compound of Claim 2, where R₁=R₂=CH₃.

3. The compound of Claim 1, which is 3-[4-[4-(benzo[b]-thien-2-yl)-1-piperidinyl]butyl]-2,5,5-trimethyl-4-thiazolidinone hydrochloride.

4. The compound of Claim 1, which is 3-[4-[4-(benzo[b]-thien-2-yl)-1-piperidinyl]butyl]-2,5,5-trimethyl-4-thiazolidinone maleate.

5. The compound of Claim 1, which is 3-[4-[4-(benzo[b]-thien-3-yl)-1-piperidinyl]butyl]-5,5-dimethyl-1,1-dioxo-4-thiazolidinone.

6. An antipsychotic composition comprising an effective psychosis alleviating amount of a compound as defined in Claim 1 and a suitable carrier therefor.

7. A method of treating a patient in need of relief from psychosis which comprises administration of an effective psychosis alleviating amount of a compound as defined in Claim 1.
